(19) Europäisches Patentamt
European Patent Office
Office européen des brevets

(11) Veröffentlichungsnummer: **0 100 478 A2**

# (12) EUROPÄISCHE PATENTANMELDUNG

(21) Anmeldenummer: 83107008.1

(22) Anmeldetag: 18.07.83

(51) Int. Cl.³: C 07 C 93/14
C 07 C 149/42, A 01 N 33/12

(30) Priorität: 31.07.82 DE 3228728

(43) Veröffentlichungstag der Anmeldung: 15.02.84 Patentblatt 84/7

(84) Benannte Vertragsstaaten: BE CH DE FR GB LI

(71) Anmelder: BAYER AG
Zentralbereich Patente, Marken und Lizenzen
D-5090 Leverkusen 1, Bayerwerk(DE)

(72) Erfinder: Kühle, Engelbert, Dr.
von-Bodelschwingh-Strasse 42
D-5060 Bergisch Gladbach 2(DE)

(72) Erfinder: Klauke, Erich, Dr.
Eichendorffweg 8
D-5068 Odenthal(DE)

(72) Erfinder: Lürssen, Klaus, Dr.
August-Kierspel-Strasse 89
D-5060 Bergisch Gladbach 2(DE)

## (54) Substituierte Benzylammonium-Salze.

(57) Neue substituierte Benzylammonium-Salze der Formel

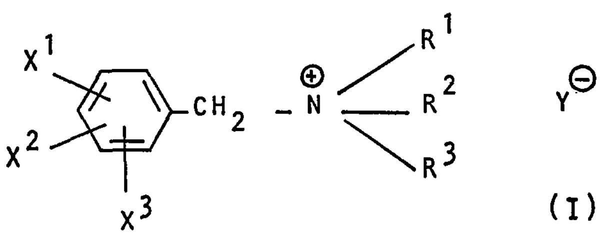

(I)

in welcher

$X^1$ für Trifluormethoxy, Fluor-dichlor-methoxy, Difluor-chlor-methoxy, Trifluor-methylthio, Fluor-dichlormethylthio, Difluor-chlor-methylthio, Trifluor-methyl-sulfinyl, Trifluor-methyl-sulfonyl, Fluor-dichlor-methyl-sulfinyl, Fluor-dichlor-methyl-sulfonyl, Difluor-chlor-methyl-sulfinyl oder Difluor-chlor-methyl-sulfonylsteht,

$X^2$ für Wasserstoff oder Halogen steht,

$X^3$ für Wasserstoff oder Halogen steht,

$R^1$, $R^2$ und $R^3$ unabhängig voneinander für Alkyl mit 1 bis 6 Kohlenstoffatomen, Alkenyl mit 2 bis 6 Kohlenstoffatomen, Alkinyl mit 2 bis 6 Kohlenstoffatomen oder Alkoxyalkyl mit 1 bis 4 Kohlenstoffatomen in der Alkoxygruppe und 1 bis 4 Kohlenstoffatomen in der Alkylgruppen stehen, oder

$R^2$ und $R^2$ gemeinsam für einen Alkylbrücke mit 4 bis 7 Kohlenstoffatomen stehen und

$Y^{\ominus}$ für Halogenid steht,

ein Verfahren zur Herstellung dieser neuen Stoffe und deren Verwendung als Pflanzenwachstumsregulatoren.

EP 0 100 478 A2

Croydon Printing Company Ltd.

0100478

BAYER AKTIENGESELLSCHAFT 5090 Leverkusen, Bayerwerk

Zentralbereich
Patente, Marken und Lizenzen Dü/Kü-c

Ia

## Substituierte Benzylammonium-Salze

Die Erfindung betrifft neue substituierte Benzylammonium-Salze, ein Verfahren zu deren Herstellung und deren Verwendung als Pflanzenwachstumsregulatoren.

Es ist bereits bekannt geworden, daß bestimmte Benzyltrialkylammonium-Salze, die eine Trifluormethylgruppe in der meta-Position des Benzylrestes enthalten, pflanzenwuchsregulierende Eigenschaften besitzen (vgl. CA-PS 1 090 799). So läßt sich zum Beispiel das (3-Trifluor-methyl-benzyl)-tri-n-butyl-ammonium-chlorid zur Beeinflussung des Pflanzenwachstums einsetzen. Die Wirkung dieses Stoffes ist jedoch, insbesondere bei niedrigen Aufwandmengen nicht immer befriedigend.

Es wurden nun neue substituierte Benzylammonium-Salze der Formel

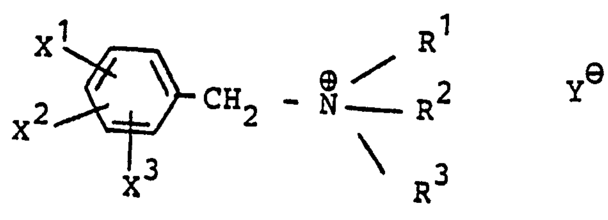

(I)

in welcher

$X^1$ für Trifluormethoxy, Fluor-dichlor-methoxy, Difluor-chlor-methoxy, Trifluor-methylthio, Fluor-dichlor-methylthio, Difluor-chlor-methylthio, Trifluor-methyl-sulfinyl, Trifluormethyl-sulfonyl, Fluor-dichlor-methyl-sulfinyl, Fluor-dichlor-methyl-sulfonyl, Difluor-chlor-methyl-sulfinyl oder Difluor-chlor-methyl-sulfonyl steht,

$X^2$ für Wasserstoff oder Halogen steht,

$X^3$ für Wasserstoff oder Halogen steht,

$R^1$, $R^2$ und $R^3$ unabhängig voneinander für Alkyl mit 1 bis 6 Kohlenstoffatomen, Alkenyl mit 2 bis 6 Kohlenstoffatomen, Alkinyl mit 2 bis 6 Kohlenstoffatomen oder Alkoxyalkyl mit 1 bis 4 Kohlenstoffatomen in der Alkoxygruppe und 1 bis 4 Kohlenstoffatomen in der Alkylgruppe stehen, oder

$R^1$ und $R^2$ gemeinsam für eine Alkylenbrücke mit 4 bis 7 Kohlenstoffatomen stehen und

$Y^{\ominus}$ für Halogenid steht,

gefunden.

Weiterhin wurde gefunden, daß man die neuen substituierten Benzylammonium-Salze der Formel (I) erhält, wenn man Benzylhalogenide der Formel

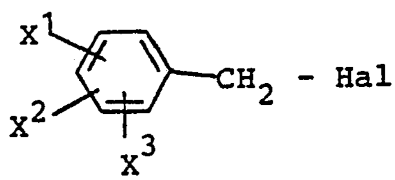

(II)

in welcher

$X^1$, $X^2$ und $X^3$ die oben angegebenen Bedeutungen haben und

Hal für Halogen steht,

mit Aminen der Formel

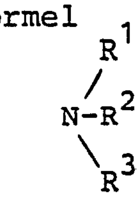

(III)

in welcher

$R^1$, $R^2$ und $R^3$ die oben angegebenen Bedeutungen haben,

gegebenenfalls in Gegenwart eines Verdünnungsmittels umsetzt.

Schließlich wurde gefunden, daß sich die neuen substituierten Benzylammonium-Salze der Formel (I) durch starke pflanzenwuchsregulierende Eigenschaften auszeichnen.

Überraschenderweise besitzen die erfindungsgemäßen substituierten Benzylammonium-Salze der Formel (I) eine bessere pflanzenwuchsregulierende Wirksamkeit als das (3-Trifluormethyl-benzyl)-tri-n-butyl-ammonium-chlorid, welches der konsitutionell ähnlichste vorbekannte Wirkstoff gleicher Wirkungsrichtung ist.

Die erfindungsgemäßen substituierten Benzylammonium-Salze sind durch die Formel (I) allgemein definiert. In dieser Formel stehen vorzugsweise

$X^1$ für Trifluormethoxy, Fluor-dichlor-methoxy, Difluor-chlor-methoxy, Trifluormethylthio, Fluor-dichlor-methylthio, Difluor-chlor-methylthio, Trifluor-methyl-sulfinyl, Trifluormethyl-sulfonyl, Fluor-dichlor-methyl-sulfinyl, Fluor-dichlor-methyl-sulfonyl, Difluor-chlor-methyl-sulfinyl oder Difluor-chlor-methyl-sulfonyl,

$X^2$ für Wasserstoff, Fluor oder Chlor,

$X^3$ für Wasserstoff, Fluor oder Chlor,

$R^1$, $R^2$ und $R^3$ unabhängig voneinander für Methyl, Ethyl, Propyl, iso-Propyl, n-Butyl, iso-Butyl, n-Pentyl, iso-Pentyl, Vinyl, Propenyl, Propargyl, Methoxymethyl, Ethoxymethyl, Methoxyethyl oder Ethoxyethyl, oder

$R^1$ und $R^2$ stehen gemeinsam für eine Alkylenbrücke mit 4 bis 6 Kohlenstoffatomen und

$Y^{\ominus}$ steht vorzugsweise für Chlorid oder Bromid.

Verwendet man beispielsweise 3-Trifluormethoxy-benzylchlorid und Tri-n-butylamin als Ausgangsstoffe, so kann der Verlauf des erfindungsgemäßen Verfahrens durch das folgende Formelschema wiedergegeben werden:

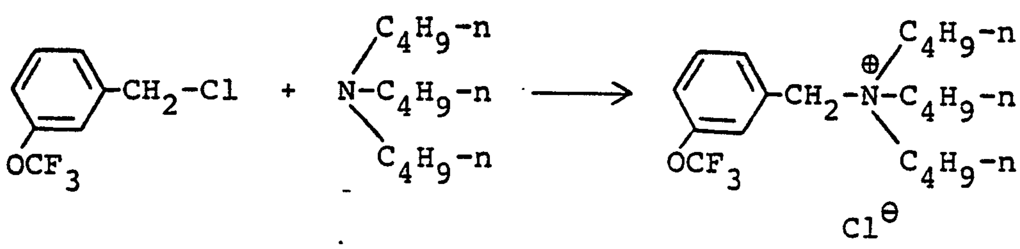

Die bei dem erfindungsgemäßen Verfahren als Ausgangsstoffe benötigten Benzylhalogenide sind durch die Formel (II) allgemein definiert. In dieser Formel haben $X^1$, $X^2$ und $X^3$ vorzugsweise diejenigen Bedeutungen, die bereits im Zusammenhang mit der Beschreibung der erfindungsgemäßen Stoffe der Formel (I) vorzugsweise für diese Reste genannt wurden. Hal steht vorzugsweise für Chlor oder Brom.

Die Benzylhalogenide der Formel (II) sind bekannt oder lassen sich in einfacher Weise nach bekannten Verfahren herstellen (vgl. DE-OS 29 05 081). So erhält man Benzylhalogenide der Formel (II), wenn man

a) Benzoylfluoride der Formel

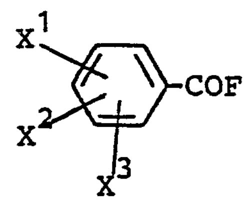

(IV)

in welcher

$X^1$, $X^2$ und $X^3$ die oben angegebenen Bedeutungen haben,

in Gegenwart eines Verdünnungsmittels mit starken Reduktionsmitteln umsetzt und die dabei entstehenden Benzylalkohole der Formel

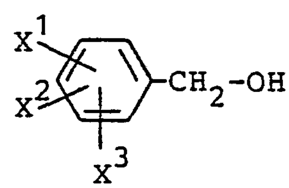

(V)

in welcher

$X^1$, $X^2$ und $X^3$ die oben angegebenen Bedeutungen haben,

gegebenenfalls in Gegenwart eines Verdünnungsmittels mit Halogenierungsmitteln umsetzt,

oder

b) Toluol-Derivate der Formel

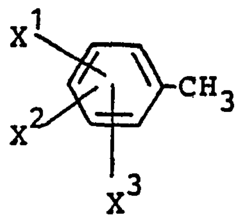

(VI)

in welcher

$X^1$, $X^2$ und $X^3$ die oben angegebenen Bedeutungen haben,

gegebenenfalls in Gegenwart eines Verdünnungsmittels mit Reagenzien, die zur Halogenierung der Methylgruppe geeignet sind, umsetzt.

Die bei dem Verfahren (a) als Ausgangsstoffe benötigten Benzoylfluoride sind durch die Formel (IV) allgemein definiert. In dieser Formel haben $X^1$, $X^2$ und $X^3$ vorzugsweise diejenigen Bedeutungen, die bereits im Zusammenhang mit der Beschreibung der erfindungsgemäßen Stoffe der Formel (I) vorzugsweise für diese Reste genannt wurden.

Die Benzoylfluoride der Formel (IV) sind bekannt oder lassen sich nach prinzipiell bekannten Methoden in einfacher Weise herstellen (vgl. DE-OS 21 29 200 und DE-OS 23 25 089).

Zur Überführung der Benzoylfluoride der Formel (IV) in Benzylalkohole der Formel (V) kommen alle für derartige Reaktionen geeigneten Reduktionsmittel in Frage. Besonders bevorzugt verwendbar ist Natriumborhydrid.

Als Verdünnungsmittel kommen bei der Durchführung der ersten Stufe des Verfahrens (a) alle für derartige Umsetzungen üblichen inerten organischen Solventien in Betracht. Vorzugsweise verwendbar sind Ether, wie Diethylether, Dioxan und Tetrahydrofuran. Ferner kann auch Wasser oder Wasser im Gemisch mit Alkoholen verwendet werden.

Die Reaktionstemperaturen können bei der Durchführung der ersten Stufe des Verfahrens (a) innerhalb eines größeren Bereiches variiert werden. Im allgemeinen arbeitet man bei Temperaturen zwischen -20°C und 120°C, vorzugsweise zwischen 20°C und 100°C.

Im einzelnen verfährt man bei der Durchführung der ersten Stufe des Verfahrens (a) in der Weise, daß man auf 1 Mol an Benzoylfluorid der Formel (IV) die stöchiometrisch benötigte Menge oder auch einen Überschuß an Reduktionsmittel einsetzt. Nach beendeter Umsetzung arbeitet man nach üblichen Methoden auf. Im allgemeinen geschieht das in der Weise, daß man das Reaktionsgemisch in Wasser gibt, dann ansäuert, die organische Phase abtrennt, trocknet und destilliert.

Bei der Durchführung der zweiten Stufe des Verfahrens (a) kommen als Halogenierungsmittel alle diejenigen Stoffe in Frage, die zur Halogenierung von Alkoholen geeignet sind. Vorzugsweise verwendbar sind Phosphortrichlorid, Phosphorpentachlorid, Phosphortribromid, Thionylchlorid und Thionylbromid.

Als Verdünnungsmittel kommen bei der Durchführung der zweiten Stufe des Verfahrens (a) alle für derartige Umsetzungen üblichen inerten organischen Solventien in Betracht. Vorzugsweise arbeitet man ohne Lösungsmittel.

Die Reaktionstemperaturen können auch bei der Durchführung der zweiten Stufe des Verfahrens (a) innerhalb eines größeren Bereiches variiert werden. Im allgemeinen arbeitet man bei Temperaturen zwischen 0°C und 120°C, vorzugsweise zwischen 10°C und 100°C.

Bei der Durchführung der zweiten Stufe des Verfahrens (a) setzt man auf 1 Mol an Benzylalkohol der Formel (V) eine äquivalente Menge oder auch einen Überschuß an Halogenierungsmittel ein. Die Aufarbeitung erfolgt nach üblichen Methoden. Im allgemeinen verfährt man in der Weise, daß man das Reaktionsgemisch nach beendeter Umsetzung vom überschüssigen Halogenierungsmittel befreit und anschließend destilliert.

Die bei dem Verfahren (b) als Ausgangsstoffe benötigten Toluol-Derivate sind durch die Formel (VI) allgemein definiert. In dieser Formel haben $X^1$, $X^2$ und $X^3$ vorzugsweise diejenigen Bedeutungen, die bereits im Zusammenhang mit der Beschreibung der erfindungsgemäßen Stoffe der Formel (I) vorzugsweise für diese Reste genannt wurden.

Die Toluol-Derivate der Formel (VI) sind bekannt oder lassen sich nach prinzipiell bekannten Methoden in einfacher Weise herstellen. So lassen sich zum Beispiel diejenigen Verbindungen der Formel (VI), in denen $X^1$ für Trifluormethyl-sulfinyl, Trifluormethyl-sulfonyl, Fluor-dichlormethyl-sulfinyl, Fluor-dichlormethyl-sulfonyl, Difluor-chlormethyl-sulfinyl oder Difluor-chlormethyl-sulfonyl steht, dadurch herstellen, daß man die zugrundeliegenden Verbindungen der Formel (VI), in denen $X^1$ für Trifluormethylthio, Fluor-dichlormethylthio oder Difluor-chlormethylthio steht, mit sauerstoffabgebenden Oxidationsmitteln, wie Wasserstoffperoxid, Peressigsäure, m-Chlorperbenzoesäure oder Chromtrioxid, in Gegenwart eines Verdünnungsmittels, wie Eisessig oder Methylenchlorid, bei Temperaturen zwischen 0°C und 120°C, vorzugsweise zwischen 50°C und 100°C umsetzt (vgl. DE-OS 27 03 477).

Als Reaktionskomponenten kommen bei der Durchführung des Verfahrens (b) alle diejenigen Stoffe in Betracht, die zur Halogenierung der Methylgruppe geeignet sind. Vorzugsweise verwendbar sind Brom oder Chlor bei gleichzeitiger Bestrahlung des Reaktionsgemisches mit UV-Licht.

Als Verdünnungsmittel kommen bei der Durchführung des Verfahrens (b) alle für derartige Umsetzungen üblichen inerten organischen Solventien in Frage. Vorzugsweise arbeitet man ohne Lösungsmittel.

Die Reaktionstemperaturen können bei dem Verfahren (b) innerhalb eines größeren Bereiches variiert werden. Im allgemeinen arbeitet man bei Temperaturen zwischen 20°C und 140°C, vorzugsweise zwischen 60°C und 100°C.

Bei der Durchführung des Verfahrens (b) setzt man auf 1 Mol an Toluol-Derivat der Formel (VI) eine äquivalente Menge oder auch einen Überschuß an Halogenierungsmittel ein. Die Aufarbeitung erfolgt nach üblichen Methoden. Im allgemeinen verfährt man in der Weise, daß man das Reaktionsgemisch nach beendeter Umsetzung einer fraktionierten Destillation unterwirft.

Die bei dem erfindungsgemäßen Verfahren zur Herstellung der substituierten Benzylammonium-Salze der Formel (I) als Reaktionskomponenten benötigten Amine sind durch die Formel (III) allgemein definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele für besonders bevorzugte Amine der Formel (III) seien die in der nachstehenden Tabelle 1 formelmäßig aufgeführten Stoffe genannt.

Tabelle 1

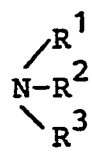

(III)

| $R^1$ | $R^2$ | $R^3$ |
|---|---|---|
| $C_2H_5$ | $C_2H_5$ | $C_2H_5$ |
| $C_3H_7$-n | $C_3H_7$-n | $C_3H_7$-n |
| $C_3H_7$-iso | $C_2H_5$ | $C_2H_5$ |
| $C_4H_9$-n | $C_4H_9$-n | $C_4H_9$-n |
| $C_5H_{11}$-n | $C_5H_{11}$-n | $C_5H_{11}$-n |
| $-CH_2-CH=CH_2$ | $CH_3$ | $CH_3$ |
| $-CH_2-C \equiv CH$ | $CH_3$ | $CH_3$ |
| $-C_2H_4-OCH_3$ | $-C_2H_4-OCH_3$ | $-C_2H_4-OCH_3$ |
| $-C_2H_4-O-C_2H_5$ | $-C_2H_4-O-C_2H_5$ | $-C_2H_5-O-C_2H_5$ |
| $-(CH_2)_4-$ | | $CH_3$ |
| $-(CH_2)_5-$ | | $CH_3$ |

Die Amine der Formel (III) sind bekannt oder lassen sich nach üblichen Methoden in einfacher Weise herstellen.

Als Verdünnungsmittel kommen bei dem erfindungsgemäßen Verfahren zur Herstellung der Stoffe der Formel (I) praktisch alle inerten organischen Solventien in Betracht. Vorzugsweise verwendbar sind polare Lösungsmittel, wie Acetonitril und Propionitril, außerdem Amide, wie Dimethylformamid, Dimethylacetamid und N-Methylpyrrolidon, darüber hinaus Dimethylsulfoxid, Tetramethylensulfon und Hexamethylphosphorsäuretriamid. Wird das Amin der Formel (III) in einem größeren Überschuß eingesetzt, so kann es gleichzeitig als Verdünnungsmittel fungieren. In diesem Fall erübrigt sich die Zugabe eines anderen Lösungsmittels.

Die Reaktionstemperaturen können bei der Durchführung des erfindungsgemäßen Verfahrens innerhalb eines größeren Bereiches variiert werden. Im allgemeinen arbeitet man bei Temperaturen zwischen 0°C und 150°C, vorzugsweise zwischen 20°C und 120°C.

Bei der Durchführung des erfindungsgemäßen Verfahrens setzt man die Reaktionskomponenten der Formeln (II) und (III) im allgemeinen in etwa äquimolaren Mengen ein. Es ist jedoch auch möglich eine der Komponenten im Überschuß zu verwenden. Die Aufarbeitung erfolgt nach üblichen Methoden. Im allgemeinen verfährt man in der Weise, daß man das Reaktionsgemisch einengt und den verbleibenden Rückstand gegebenenfalls nach vorhergehendem Digerieren mit einem organischen Lösungsmittel umkristallisiert.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff

auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinfluß werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in andere Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z.B. Ananas oder Zierpflanzen in Gärtnereien, zu einem Zeitpunkt keimen, austreiben oder blühen, an dem sie normalerweise hierzu

keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren

auszubringen oder die Wirkstoffzubereitung oder den Wirkstoff selbst in den Boden zu injizieren. Es kann auch das Saatgut der Pflanzen behandelt werden.

Die Aufwandmengen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Stoffe geht aus den nachfolgenden Beispielen hervor.

Herstellungsbeispiele

Beispiel 1

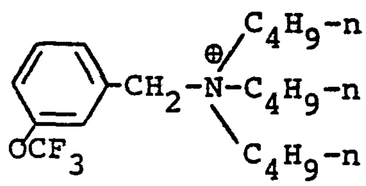

$Cl^{\ominus}$

10,5 g (50 mMol) 3-Trifluormethoxy-benzylchlorid und 9,3 g (50 mMol) Tri-n-butylamin werden in 100 ml Acetonitril gelöst und 120 Stunden unter Rückfluß erhitzt. Anschließend wird das Lösungsmittel unter vermindertem Druck abgezogen und der verbleibende Rückstand mit Diethylether behandelt. Dabei bildet sich ein kristallines Produkt, das zur Reinigung in Aceton gelöst und durch Zugabe von Petrolether ausgefällt wird. Dieses Produkt wird abfiltriert und getrocknet. Man erhält auf diese Weise 10 g (50 % der Theorie) an (3-Trifluormethoxy-benzyl)-tri-n-butylammonium-chlorid in Form einer Festsubstanz vom Schmelzpunkt 169 - 170°C.

Nach der im Beispiel 1 angegebenen Methode werden auch die in der folgenden Tabelle 2 formelmäßig aufgeführten Stoffe hergestellt.

Tabelle 2

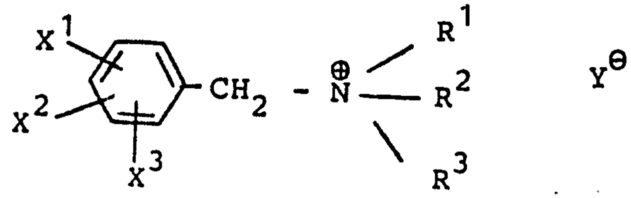

(I)

| Bsp. Nr. | $X^1$ | $X^2$ | $X^3$ | $R^1$ | $R^2$ | $R^3$ | Y | Schmelzpunkt [°C] |
|---|---|---|---|---|---|---|---|---|
| 2 | $4-F_3CO-$ | 3-Cl | H | $-C_4H_9-n$ | $-C_4H_9-n$ | $-C_4H_9-n$ | Cl | 195 - 196 |
| 3 | $4-F_3CS-$ | H | H | $-C_4H_9-n$ | $-C_4H_9-n$ | $-C_4H_9-n$ | Cl | 178 - 179 |
| 4 | $4-ClF_2CO$ | H | H | $-C_4H_9-n$ | $-C_4H_9-n$ | $-C_4H_9-n$ | Cl | 157 |
| 5 | $4-ClF_2CS$ | H | H | $-C_4H_9-n$ | $-C_4H_9-n$ | $-C_4H_9-n$ | Cl | 158 |

## Herstellung von Ausgangssubstanzen

### Beispiel (II-1)

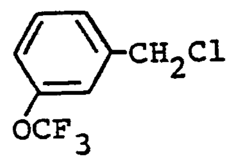

(II-1)

165 g Thionylchlorid werden vorgelegt und hierzu in 20 Minuten bei Raumtemperatur 90 g 3-Trifluormethoxibenzylalkohol zugegeben. Nach Ende der Zugabe gibt man zur Beschleunigung der Umsetzung noch 5 Tropfen Dimethylformamid zu und läßt ausreagieren. Nach Stehen über Nacht wird destilliert.

Man erhält 39 g 3-Trifluormethoxybenzylchlorid

Kp: 65°C/16 mbar

$n_D^{20}$ : 1,4537

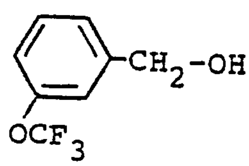

(V-1)

In ein Gemisch aus 31 g (0,8 Mol) Natriumborhydrid in 800 ml Dioxan werden bei Rückflußtemperatur innerhalb von 8 Stunden 208 g (1 Mol) 3-Trifluormethoxy-benzoylfluorid gegeben. Man rührt 1 h bei 100°C nach, kühlt auf Raumtemperatur ab, gießt das Reaktionsgemisch auf Wasser und säuert mit verdünnter Salzsäure an. Die organische Phase wird mit Methylenchlorid aufgenommen, abgetrennt, über Natriumsulfat getrocknet und destilliert. Man erhält auf diese Weise 153 g (80 % der Theorie) an 3-Trifluormethoxy-benzylalkohol.

Kp = 94°C/18 mbar

$n_D^{20}$ = 1,4496

<u>Beispiel (II-2)</u>

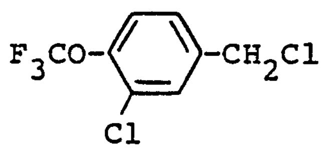

(II-2)

Kp = 100°C/18 mbar

$n_D^{20}$ = 1,4788

<u>Beispiel (II-3)</u>

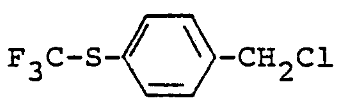

(II-3)

Kp = 97-98°C/18 mbar

$n_D^{20}$ = 1,5072

0100478

Beispiel (II-4)

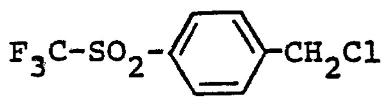

(II-4)

Kp = 144-145°C/17 mbar

$n_D^{20}$ = 1,4978

Nach den in den vorausgehenden Beispielen angegebenen Methoden werden auch die in der folgenden Tabelle 3 formelmäßig aufgeführten Ausgangssubstanzen hergestellt.

Tabelle 3

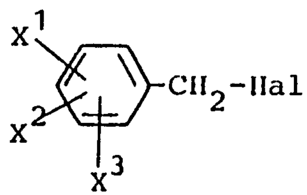

(II)

| Bsp.-Nr. | $X^1$ | $X^2$ | $X^3$ | Hal | Siedepunkt (°C) | Brechungsindex $n_D^{20}$ |
|---|---|---|---|---|---|---|
| (II-5) | 4-$ClF_2CO$- | H | H | Cl | 95/16 mbar | 1,4898 |
| (II-6) | 4-$ClF_2CS$- | H | H | Cl | 114/20 mbar | 1,5393 |
| (II-7) | 4-$F_3CO$- | H | H | Cl | 185 | 1,3439 |
| (II-8) | 4-$F_3CO$- | H | H | Br | 88/20 mbar | 1,4305 |

In den folgenden Verwendungsbeispielen wird die nachstehend angegebene Verbindung als Vergleichssubstanz eingesetzt:

(A) = 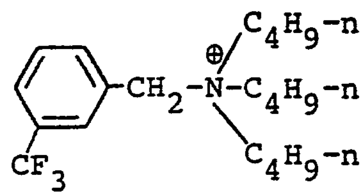 $Cl^{\ominus}$

(3-Trifluormethyl-benzyl)-tri-n-butyl-ammoniumchlorid
(bekannt aus CA-PS 1 090 799).

Beispiel A

Wuchshemmung bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

In diesem Test zeigt die erfindungsgemäße Verbindung (1) eine wesentlich stärkere wuchshemmende Wirksamkeit als die Vergleichssubstanz (A).

Beispiel B

Stimulierung der $CO_2$-Fixierung bei Sojabohnen

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Im weiteren Verlauf des Experimentes wird die $CO_2$-Fixierung der Pflanzen mit üblichen Methoden gemessen. Die Werte werden mit denen nicht mit Wirkstoffen behandelter Kontrollpflanzen verglichen.

Es bedeuten:

- \- Hemmung der $CO_2$-Fixierung
- 0 $CO_2$-Fixierung wie bei der Kontrolle
- \+ geringe Stimulierung der $CO_2$-Fixierung
- ++ starke Stimulierung der $CO_2$-Fixierung
- +++ sehr starke Stimulierung der $CO_2$-Fixierung

In diesem Test bewirkt die erfindungsgemäße Verbindung (2) eine starke Stimulierung der $CO_2$-Fixierung, während die Vergleichssubstanz (A) keine Wirkung zeigte.

Patentansprüche

1) Substituierte Benzylammonium-Salze der Formel

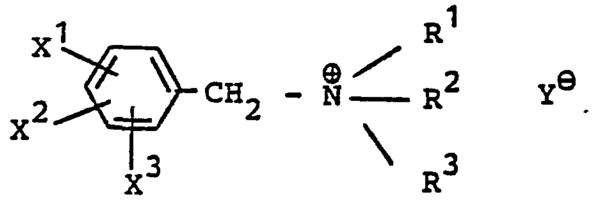

(I)

in welcher

$X^1$ für Trifluormethoxy, Fluor-dichlor-methoxy, Difluor-chlor-methoxy, Trifluormethylthio, Fluor-dichlor-methylthio, Difluor-chlor-methylthio, Trifluormethyl-sulfinyl, Trifluormethyl-sulfonyl, Fluor-dichlor-methyl-sulfinyl, Fluor-dichlor-methyl-sulfonyl, Difluor-chlor-methyl-sulfinyl oder Difluor-chlor-methyl-sulfonyl steht,

$X^2$ für Wasserstoff oder Halogen steht,

$X^3$ für Wasserstoff oder Halogen steht,

$R^1$, $R^2$ und $R^3$ unabhängig voneinander für Alkyl mit 1 bis 6 Kohlenstoffatomen, Alkenyl mit 2 bis 6 Kohlenstoffatomen, Alkinyl mit 2 bis 6 Kohlenstoffatomen oder Alkoxyalkyl mit 1 bis 4 Kohlenstoffatomen in der Alkoxygruppe und 1 bis 4 Kohlenstoffatomen in der Alkylgruppe stehen, oder

$R^1$ und $R^2$ gemeinsam für eine Alkylenbrücke mit 4 bis 7 Kohlenstoffatomen stehen und

$Y^{\ominus}$ für Halogenid steht.

2) Substituierte Benzylammonium-Salze der Formel (I), in denen

$X^1$ für Trifluormethoxy, Fluor-dichlor-methoxy, Difluor-chlor-methoxy, Trifluor-methylthio, Fluor-dichlor-methylthio, Difluor-chlor-methylthio, Trifluor-methyl-sulfinyl, Trifluor-methylsulfonyl, Fluor-dichlor-methyl-sulfinyl, Fluordichlor-methyl-sulfonyl, Difluor-chlor-methylsulfinyl oder Difluor-chlor-methyl-sulfonyl steht,

$X^2$ für Wasserstoff, Fluor oder Chlor steht,

$X^3$ für Wasserstoffe, Fluor oder Chlor steht,

$R^1$, $R^2$ und $R^3$ unabhängig voneinander für Methyl, Ethyl, Propyl, iso-Propyl, n-Butyl, iso-Butyl, n-Pentyl, iso-Pentyl, Vinyl, Propenyl, Propargyl, Methoxymethyl, Ethoxymethyl, Methoxyethyl oder Ethoxyethyl stehen oder

$R^1$ und $R^2$ gemeinsam für eine Alkylenbrücke mit 4 bis 6 Kohlenstoffatomen stehen und

$Y^{\ominus}$ für Chlorid oder Bromid steht.

3) Verfahren zur Herstellung von substituierten Benzylammonium-Salzen der Formel

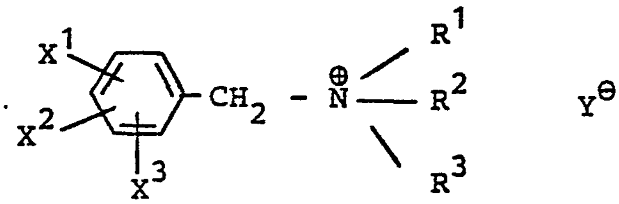

(I)

in welcher

$X^1$ für Trifluormethoxy, Fluor-dichlor-methoxy, Difluor-chlor-methoxy, Trifluor-methylthio, Fluor-dichlor-methylthio, Difluor-chlor-methylthio, Trifluor-methyl-sulfinyl, Trifluor-methylsulfonyl, Fluor-dichlor-methyl-sulfinyl, Fluor-dichlor-methyl-sulfonyl, Difluor-chlor-methylsulfinyl oder Difluor-chlor-methyl-sulfonyl steht,

$X^2$ für Wasserstoff oder Halogen steht,

$X^3$ für Wasserstoff oder Halogen steht,

$R^1$, $R^2$ und $R^3$ unabhängig voneinander für Alkyl mit 1 bis 6 Kohlenstoffatomen, Alkenyl mit 2 bis 6 Kohlenstoffatomen, Alkinyl mit 2 bis 6 Kohlenstoffatomen oder Alkoxyalkyl mit 1 bis 4 Kohlenstoffatomen in der Alkoxygruppe und 1 bis 4 Kohlenstoffatomen in der Alkylgruppe stehen, oder

$R^1$ und $R^2$ gemeinsam für eine Alkylenbrücke mit 4 bis 7 Kohlenstoffatomen stehen und

$Y^{\ominus}$ für Halogenid steht,

dadurch gekennzeichnet, daß man Benzylhalogenide der Formel

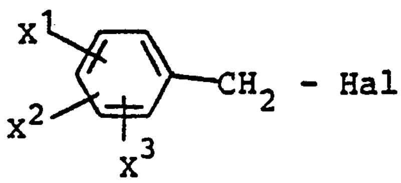

(II)

in welcher

$X^1$, $X^2$ und $X^3$ die oben angegebenen Bedeutungen haben und

Hal für Halogen steht,

mit Aminen der Formel

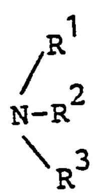

(III)

in welcher

$R^1$, $R^2$ und $R^3$ die oben angegebenen Bedeutungen haben,

gegebenenfalls in Gegenwart eines Verdünnungsmittels umsetzt.

4) Pflanzenwuchsregulierende Mittel, gekennzeichnet durch einen Gehalt an mindestens einem substituierten Benzylammonium-Salz der Formel (I).

5) Verfahren zur Regulierung des Pflanzenwachstums, dadurch gekennzeichnet, daß man substituierte Benzylammonium-Salze der Formel (I) auf die Pflanzen und/oder deren Lebensraum ausbringt.

6) Verwendung von substituierten Benzylammonium-Salzen der Formel (I) zur Regulierung des Pflanzenwachstums.

7) Verfahren zur Herstellung von pflanzenwuchsregulierenden Mitteln, dadurch gekennzeichnet, daß man substituierte Benzylammonium-Salze der Formel (I) mit Streckmitteln und/oder oberflächenaktiven Stoffen vermischt.

8) Substituiertes Benzylammonium-Salz der Formel

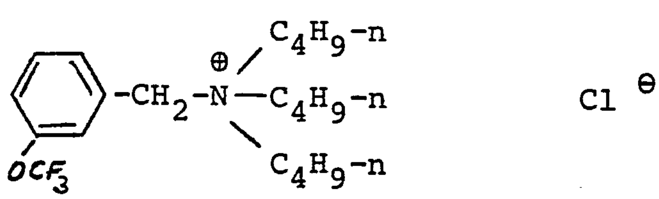

9) Substituiertes Benzylammonium-Salz der Formel

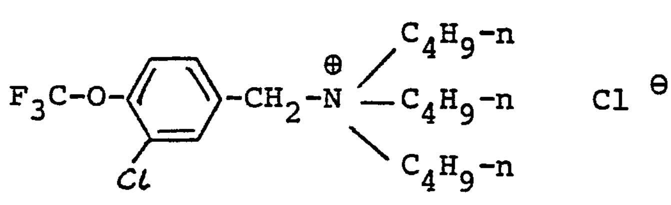

10) Substituiertes Benzylammonium-Salz der Formel